# EUROPEAN PATENT APPLICATION

(11) **EP 4 714 422 A1**
(43) Date of publication of application: **25.03.2026**
(21) Application number: 25203316.2
(22) Date of filing: 19.09.2025
(51) Int. Cl.: A61K 8/19, A61K 8/36, A61K 8/73, A61Q 1/00, A61Q 1/12

(54) **COMPOSITION FOR ABSORBING SKIN PERSPIRATION AND/OR BODY MOISTURE**

(30) Priority: 20.09.2024 IT 202400021067
(71) Applicant: Società Italo Britannica L. Manetti - H. Roberts & C.p.A., 50123 Firenze (IT)
(72) Inventor: SANTI, Maurizio, 59100 Prato (PO) (IT); TARGETTI, Andrea, 59100 Prato (PO) (IT); RESCIGNANO, Dalila, 59100 Prato (PO) (IT)
(74) Representative: Bird & Bird Società tra Avvocati S.r.l.

(57) **Abstract**

The present invention relates to an absorbent composition, the use of said composition for the absorption of skin perspiration and/or body moisture, a non-therapeutic cosmetic method comprising a step for applying said composition to the skin, the topical use of said composition in the prevention and/or treatment of inflammatory skin conditions and a process for the production of said composition.

## Description

The present invention relates to an absorbent composition, the use of said composition for the absorption of skin perspiration and/or body moisture, a non-therapeutic cosmetic method comprising a step for applying said composition to the skin, the topical use of said composition in the prevention and/or treatment of inflammatory skin conditions and a process for the production of said composition.

### FIELD OF THE INVENTION AND RELATIVE STATE OF THE ART

The present invention falls within the technical sector of compositions suitable for absorbing skin perspiration and/or body moisture.

As is well known, there are various pharmaceutical and/or cosmetic products on the market capable of exerting these functions. In particular, talc has been used for many years in powder form for application to the skin. Talc has particularly interesting properties, including: high softness (it has a value of 1 on the Mohs scale); the presence of magnesium phyllosilicate with a tetrahedral structure; a lamellar structure; a greasy feel; good anti-adhesive properties; a hydrophobic and anhydrous nature; adequate flowability; good ability for absorbing lipophilic substances; acceptable colour; and low production costs.

Thanks to these characteristics, talc has found wide application in the cosmetic industry for its absorbent, refreshing and velvety properties, in particular for the treatment of delicate skin, such as that of newborn babies and children, to prevent irritation.

The geological formation process of talc however has various challenges, as the chemical-physical processes, temperatures, and pressures involved can lead to the formation of other fibrous minerals. These fibrous formations (asbestos) must be absolutely avoided and monitored to ensure the safety of the talc. Choosing a safe source of talc is essential for ensuring the absence of asbestos and, at the same time, a low heavy metal content.

Furthermore, inhaling talc powder is known to cause respiratory problems, especially in children and people with pre-existing respiratory conditions.

It is also important to note that the annual rate of talc consumption exceeds the rate of mineral formation, effectively constituting an unsustainable long-term supply.

In this context, various alternative formulations have been developed with the aim of addressing (or at least limiting) the disadvantages associated with the use of talc. These formulations typically include starch and a glidant agent that prevents it from caking. These formulations, however, have demonstrated objective limitations in terms of water-repellent and/or absorbent properties with respect to talc. In particular, starches have absorbent properties, but are unable to satisfactorily absorb oily substances and/or saline solutions (such as sweat). Furthermore, the glidant agents used in these formulations have limitations in terms of toxicity.

There is therefore a particular need in the industry for providing formulations capable of solving the disadvantages associated with the use of talc while guaranteeing the same performance in terms of water-repellent and absorbent properties that characterize this product.

### SUBJECT OF THE INVENTION

In order to overcome the above disadvantages of the prior art, a composition has been developed comprising or consisting of:
a) A filling agent consisting of starch, most preferably rice starch, corn starch or mixtures thereof;
b) A glidant agent, said glidant agent preferably being selected from magnesium carbonate hydroxide, magnesium hydroxide, magnesium carbonate, tricalcium phosphate, silica, lauroyl lysine and mixtures thereof, said glidant agent being even more preferably magnesium carbonate hydroxide;
c) An absorbing agent consisting of cellulose, microcrystalline cellulose, methylcellulose, methoxycellulose, hydroxyethylcellulose, hydroxypropylcellulose or mixtures thereof;
d) A hydrophobic agent consisting of a salt of a fatty acid or a mixture of salts of fatty acids, more preferably said hydrophobic agent being a calcium salt of a fatty acid, even more preferably calcium stearate;
e) Optionally, a perfuming agent.

The present invention also relates to the use of said composition for the absorption of skin perspiration and/or body moisture (wherein said composition is applied topically).

The present invention also provides for the topical use of said composition in the prevention and/or treatment of inflammatory skin conditions, more preferably in the prevention and/or treatment of dermatitis, even more preferably contact dermatitis.

The present invention also relates to a non-therapeutic cosmetic method comprising a step for applying said composition to the skin. For the purposes of this document, "cosmetic method" means the application of said composition to the skin (or in any case to the external surfaces of the human body) for the purpose of modifying its appearance and/or maintaining it in good condition.

In particular, said method can be used for absorbing skin perspiration and/or body moisture.

The invention also relates to a process for producing said composition.

### DETAILED DESCRIPTION OF THE INVENTION

As previously specified, a composition has been developed comprising or consisting of:
a) A filling agent consisting of starch, most preferably rice starch, corn starch or mixtures thereof;
b) A glidant agent, said glidant agent preferably being selected from magnesium carbonate hydroxide, magnesium hydroxide, magnesium carbonate, tricalcium phosphate, silica, lauroyl lysine and mixtures thereof, said glidant agent being even more preferably magnesium carbonate hydroxide;
c) An absorbing agent consisting of cellulose, microcrystalline cellulose, methylcellulose, methoxycellulose, hydroxyethylcellulose, hydroxypropylcellulose or mixtures thereof;
d) A hydrophobic agent consisting of a salt of a fatty acid or a mixture of salts of fatty acids, said hydrophobic agent more preferably being a calcium salt of a fatty acid, even more preferably calcium stearate;
e) Optionally, a perfuming agent.

It has in fact been surprisingly found that this composition is characterized by performances in terms of water-repellent and absorbent properties comparable (or even superior) to those of talc, at the same time without having the disadvantages of this product. In particular, the composition according to the present invention is characterized by the complete absence of fibrous formations (asbestos) and, consequently, by a better safety profile with respect to talc.

The composition according to the present invention is also characterized by a high versatility. This is because it is possible:
- to easily modulate the hydrophobic nature of the composition by modifying the quantity of the hydrophobic agent and/or
- to easily modulate the absorbent power of the composition by changing the quantity of the absorbing agent.

In a preferred embodiment:
- said filling agent is present in a concentration by weight with respect to the total weight of the composition ranging from 50 to 90%;
- said glidant agent is present in a concentration by weight with respect to the total weight of the composition ranging from 5 to 20%;
- said absorbing agent is present in a concentration by weight with respect to the total weight of the composition ranging from 0.5 to 45% and
- said hydrophobic agent is present in a concentration by weight with respect to the total weight of the composition ranging from 0.5 to 5%.

In a further preferred embodiment:
- said filling agent is present in a concentration by weight with respect to the total weight of the composition ranging from 50 to 85%;
- said glidant agent is present in a concentration by weight with respect to the total weight of the composition ranging from 10 to 15%, more preferably equal to 13%;
- said absorbing agent is present in a concentration by weight with respect to the total weight of the composition ranging from 0.5 to 40% and
- said hydrophobic agent is present in a concentration by weight with respect to the total weight of the composition ranging from 1 to 3%, more preferably equal to 2%.

In a further preferred embodiment:
- said filling agent is present in a concentration by weight with respect to the total weight of the composition ranging from 50 to 60%;
- said glidant agent is present in a concentration by weight with respect to the total weight of the composition ranging from 10 to 15%;
- said absorbing agent is present in a concentration by weight with respect to the total weight of the composition ranging from 25 to 40% and
- said hydrophobic agent is present in a concentration by weight with respect to the total weight of the composition ranging from 0.5 to 5%.

Said filling agent can be either a modified or native starch, preferably heat-treated.

This is because native starches are soluble when hot but insoluble when cold and by their nature are hygroscopic and have a residual moisture content greater than 7% (even when heat-treated). This parameter is important as it can negatively impact the flowability of the composition, leading to a greater propensity for caking and bacterial contamination.

In one embodiment, said starch is rice starch or corn starch. Alternatively, a mixture of rice starch and corn starch can be used. The weight ratio of rice starch with respect to corn starch in said mixture can range from 0.1 to 10, more preferably from 0.5 to 4, and even more preferably from 1 to 1.5.

Said filling agent can be present in a concentration by weight with respect to the total weight of the composition ranging from 50 to 90%.

In a preferred embodiment, said absorbing agent is present in a concentration by weight with respect to the total weight of the composition ranging from 0.5 to 45%, more preferably from 5 to 40% (even more preferably from 10 to 35%).

In a preferred embodiment, said absorbing agent is cellulose. Cellulose in fact has some characteristics that are useful for the composition according to the present invention. In particular:
- it has a capillary fibrous nature which contributes significantly to the absorbent capacity of the composition;
- it is linked to low costs and high sustainability;
- it is insoluble in both hot and cold water, thus preventing agglomeration phenomena.

Said hydrophobic agent can consist of a salt (such as a calcium salt, a magnesium salt or a potassium salt) of a fatty acid or a mixture of fatty acid salts, such as salts of saturated fatty acids such as stearic acid, palmitic acid, myristic acid and arachidic acid.

Said hydrophobic agent can consist, for example, of calcium stearate, magnesium stearate or mixtures thereof, more preferably said hydrophobic agent consists of calcium stearate.

The presence of said hydrophobic agent increases the lipophilic nature of the composition, preventing it from rapidly getting wet and triggering the processing phenomenon of the starch (with consequent agglomeration).

Furthermore, the presence of said hydrophobic agent allows the quantity of glidant agent to be limited. This feature is important as glidant agents can have NOAEL values (No Observed Adverse Effect Level, a parameter used in toxicology for expressing the maximum dose of a xenobiotic that can be administered without detecting toxic effects), which do not allow the use of this ingredient in large quantities.

Said hydrophobic agent is present in a quantity that does not cause the composition to become excessively sticky to the skin (resulting in difficulty in removing residues of the composition, for example, from the hands). Said hydrophobic agent can be present, for example, in a concentration by weight with respect to the total weight of the composition ranging from 0.1 to 10%, more preferably from 0.5 to 5%, and even more preferably from 1 to 3% (for example, a concentration by weight equal to 2%).

As previously stated, said glidant agent is selected from magnesium carbonate hydroxide, magnesium hydroxide, magnesium carbonate, tricalcium phosphate, silica, lauroyl lysine and mixtures thereof, said glidant agent being even more preferably magnesium carbonate hydroxide.

Said glidant agent can be present in a concentration by weight with respect to the total weight of the composition ranging from 5 to 20%, more preferably from 10 to 15%, even more preferably equal to 13%.

Said perfuming agent can, for example, be a fragrance composition of either a synthetic or natural origin, or even natural essential oils. The fragrance composition can consist of more persistent base notes, central notes that define the olfactory characteristics of the perfume, and more volatile top notes, possibly citrus, that enhance its freshness.

Said perfuming agent can be present in a concentration by weight with respect to the total weight of the composition ranging from 0.01 to 5%, more preferably from 0.1 to 0.5%.

In one embodiment:
- said filling agent consists of a mixture of rice starch and corn starch, more preferably the weight ratio between rice starch and corn starch ranges from 0.5 to 4 (even more preferably the weight ratio between rice starch and corn starch ranges from 1 to 1.5), and/or
- said glidant agent is magnesium carbonate hydroxide.

In one embodiment:
- said glidant agent is magnesium carbonate hydroxide;
- said absorbing agent is cellulose and
- said water-repellent agent is calcium stearate.

In one embodiment:
- said filling agent consists of a mixture of rice starch and corn starch, more preferably the weight ratio between rice starch and corn starch ranges from 1 to 1.5;
- said glidant agent is magnesium carbonate hydroxide;
- said absorbing agent is cellulose and
- said water-repellent agent is calcium stearate.

In one embodiment:
- said filling agent consists of a mixture of rice starch and corn starch wherein the weight ratio between rice starch and corn starch ranges from 0.5 to 4 (even more preferably the weight ratio between rice starch and corn starch ranges from 1 to 1.5) and is present in a concentration by weight with respect to the total weight of the composition ranging from 50 to 90%,
- said glidant agent is magnesium carbonate hydroxide and is present in a concentration by weight with respect to the total weight of the composition ranging from 5 to 20%,
- said absorbing agent is cellulose and is present in a concentration by weight with respect to the total weight of the composition ranging from 0.5 to 45% and
- said hydrophobic agent is calcium stearate and is present in a concentration by weight with respect to the total weight of the composition ranging from 0.5 to 5%.

In one embodiment:
- said filling agent consists of a mixture of rice starch and corn starch wherein the weight ratio between rice starch and corn starch ranges from 0.5 to 4 (even more preferably the weight ratio between rice starch and corn starch ranges from 1 to 1.5) and is present in a concentration by weight with respect to the total weight of the composition ranging from 50 to 85%,
- the glidant agent is magnesium carbonate hydroxide and is present in a concentration by weight with respect to the total composition ranging from 10 to 15%, preferably 13%,
- the absorbing agent is cellulose and is present in a concentration by weight with respect to the total composition ranging from 0.5 to 40% and
- said hydrophobic agent is calcium stearate and is present in a concentration by weight with respect to the total weight of the composition ranging from 1 to 3%, preferably equal to 2%.

In one embodiment:
- said filling agent consists of a mixture of rice starch and corn starch wherein the weight ratio between rice starch and corn starch ranges from 0.5 to 4 (even more preferably the weight ratio between rice starch and corn starch ranges from 1 to 1.5) and is present in a concentration by weight with respect to the total weight of the composition ranging from 50 to 60%,
- said glidant agent is magnesium carbonate hydroxide and is present in a concentration by weight with respect to the total weight of the composition ranging from 10 to 15%, and is preferably equal to 13%,
- said absorbing agent is cellulose and is present in a concentration by weight with respect to the total weight of the composition ranging from 25 to 40% and
- said hydrophobic agent is calcium stearate and is present in a concentration by weight with respect to the total weight of the composition ranging from 1 to 3%, and is preferably equal to 2%.

The composition according to the present invention can have a bulk density equal to or less than 0.4 g/ml, more preferably equal to or less than 0.33 g/ml. Said composition even more preferably has a bulk density ranging from 0.4 to 0.2 g/ml.

The bulk density of a powder is the ratio of the mass of an uncompacted powder sample with respect to its volume, including the contribution of the interparticle void space. The bulk density therefore depends on both the density of the powder particles and on their spatial arrangement within the powder layer. In accordance with one of the methodologies described in European Pharmacopoeia (section 2.9.34. *"bulk density and tapped density of powders",* method 1: measurement in a graduated cylinder), these bulk density values are measured by gently introducing into a 100 ml graduated cylinder which allows reading to 1 ml, without compacting, approximately 100 g (m) of the sample under examination (weighed with an accuracy of 0.1%). If necessary, carefully levelling the powder without compacting it and reading the apparent volume (V₀) of the unsettled powder using the nearest graduated unit. Calculating the bulk density in grams per milliliter using the formula m/V₀. The bulk density is expressed in grams per milliliter (1 g/ml = 1000 kg/m³), as the measurements are effected using graduated cylinders.

The bulk density values indicated above therefore correspond to apparent volume (V₀) values equal to or greater than 2.5 ml/g, more preferably equal to or greater than 3 ml/g. Said apparent volume even more preferably ranges from 2.5 to 5 ml/g.

The composition can also comprise other ingredients known in the art, such as additional absorbing agents, refreshing agents, antimicrobial agents, antioxidant agents and/or colouring agents.

The present invention also relates to the use of said composition for the absorption of skin perspiration and/or body moisture (wherein said composition is applied topically).

The present invention further relates to the topical use of said composition in the prevention and/or treatment of pathological (and in particular inflammatory) skin conditions and, more preferably, in the prevention and/or treatment of dermatitis, even more preferably contact dermatitis. In particular, contact dermatitis refers to an inflammation of the skin resulting from certain chemical and physical stimuli.

Said stimuli can be represented, for example, by sweat (sweat dermatitis) or by prolonged contact of the skin with substances such as feces and urine. Further stimuli can be represented, for example, by irritants such as detergents or solvents.

The composition according to the present invention can also be used for the prevention and/or treatment of chafing. The composition has a soothing effect on chafing.

The composition according to the present invention can also be used for preventing and/or treating prickly heat (i.e., an inflammation of the skin caused by obstruction of the sweat glands and the subsequent retention of sweat).

The composition according to the present invention can be further used for absorbing sebum (for example, excess sebum present in the hair or other areas of the body). In this way, it is possible to limit the proliferation of Malassezia yeasts, which are the main cause of the flaking and inflammation that characterize seborrheic dermatitis (excess sebum is in fact a factor that favours the proliferation of these yeasts).

The present invention also relates to a non-therapeutic cosmetic method comprising an application step of said composition to (intact) skin. Specifically, said method can be used for absorbing skin perspiration and/or body moisture. Said composition can be used for example in antiperspirant and/or deodorant products (particularly solid products or liquid products in which the composition according to the invention can be suspended), for example in underarm deodorants..

The invention further relates to a process for producing said composition. In particular, the filling agent, the glidant agent, the absorbing agent, the hydrophobic agent, and any additional components are mixed together until a homogeneous composition is obtained.

Said composition can be produced, for example, by mixing the filling agent, the glidant agent, the absorbing agent, the hydrophobic agent and any other additional ingredients in a mixer for a period of time ranging from 1 to 20 minutes, more preferably equal to 3 minutes.

The mixing step can be carried out using industry-standard equipment, such as a horizontal paddle mixer for powders.

A perfuming agent can also be added after the first mixing step, followed by further mixing for a period of time ranging from 1 to 20 minutes, preferably 5 minutes.

### EXAMPLES

### Example 1 - comparison of the properties of compositions according to the present invention with compositions of the state of the art

In order to evaluate the absorbent properties of compositions according to the present invention, comparative tests were carried out by analyzing the compositions according to the present invention indicated in Table 1 with the samples according to the prior art A-D.

**Table 1 - compositions according to the present invention**

| | **Sample 1** | **Sample 2** | **Sample 3** | **Sample 4** |
|---|---|---|---|---|
| **Ingredient** | **Concentration by weight (% w/w)** | **Concentration by weight (% w/w)** | **Concentration by weight (% w/w)** | **Concentration by weight (% w/w)** |
| Rice starch (Oryza sativa starch) | 52.00 | 39.65 | 30.00 | 45.00 |
| Corn starch (Zea Mays Starch) | 17.65 | 35.00 | 24.65 | 38.65 |
| Magnesium carbonate hydroxide | 13.00 | 13.00 | 13.00 | 13.00 |
| Cellulose | 15.00 | 10.00 | 30.00 | 1.00 |
| Calcium stearate | 2.00 | 2.00 | 2.00 | 2.00 |
| PERFUMING COMPOSITION | 0.35 | 0.35 | 0.35 | 0.35 |

These compositions were obtained by mixing rice starch, corn starch, magnesium carbonate hydroxide, cellulose, and calcium stearate for 3 minutes using a horizontal paddle mixer with a variable speed ranging from 10 to 100 rpm. The perfuming agent was then added. The mixture was finally further mixed for five minutes.

The samples A-D according to the prior art used for the test are indicated hereunder:
- Sample A: Commercial product "Johnson&Johnson - Johnson's Cornstarch Powder" - composition consisting of: Zea Mays (corn) starch, tricalcium phosphate, perfume.
- Sample B: "Alkagin Intimate Powder" - composition consisting of: Zea Mays (corn) starch, Triticum vulgare (wheat) starch, Oryza sativa (rice) starch, zinc oxide, tapioca starch, hydrated silica, magnesium stearate, propylene glycol, aldioxa, calendula officinalis flower extract, malva sylvestris (mallow) extract, Tilia tomentosa extract, ethyl linoleate, water, methylparabens, sodium dehydroacetate, perfume.
- Sample C: Commercial product "Talcamid" - composition consisting of: Zea Mays starch, Oryza Sativa starch, Calcium starch octanylsuccinate, Magnesium stearate, Silica, Lactic acid, Calcium lactate.
- Sample D: Commercial product "EuPhidra AmidoMio Polvere Finissima" - composition consisting of: Oryza Sativa starch (rice), aluminium starch octenylsuccinate, zinc oxide, magnesium stearate, silica.

In particular, the water, oil, and sweat absorption capacities were analyzed. These properties were measured according to the method standardized by ISO 787-*5:1980. ("Determination of oil absorption value").*

The method is based on adding linseed oil from a burette to the sample being analyzed. After each addition, the oil is rubbed into the product until conglomerates are formed. The addition of oil is stopped when a soft paste is formed. The result is expressed in grams of oil per 100 g of product.

The same method was also applied for measuring water and sweat.

**Table 2 - Experimental results of the comparative tests**

| **Sample** | **Water absorption** | **Oil absorption** | **Sweat absorption** | **Apparent volume ml/g** | **Bulk density g/ml** |
|---|---|---|---|---|---|
| Sample 1 (composition according to the invention) | 88.10 | 72.10 | 118.2 | 3.1 | 0.32 |
| Sample 2 (composition according to the invention) | 84.20 | 67.00 | 119 | 3.1 | 0.32 |
| Sample 3 (composition according to the invention) | 94 | 73 | 148.2 | 3.2 | 0.31 |
| Sample 4 (composition according to the invention) | 76.8 | 71.8 | 114.1 | 3.0 | 0.33 |
| Perfumed talc (composition according to the prior art) | 32 | 35 | 72.6 | 2.2 | 0.45 |
| Sample A (composition according to the prior art) | 63.6 | 37 | 88.9 | 1.5 | 0.67 |
| Sample B (composition according to the prior art) | 58.78 | 35.91 | 93.3 | 1.8 | 0.56 |
| Sample C (composition according to the prior art) | 58.2 | 54.6 | 84.3 | 2.3 | 0.43 |
| Sample D (composition according to the prior art) | 79.5 | 34.25 | 113.5 | 1.9 | 0.53 |

As can be seen from Table 2, samples 1-4 (i.e., the compositions according to the present invention) have a significantly higher sweat-absorbing capacity than prior art compositions (including perfumed talc). It can also be observed that as the cellulose concentration increases, the specific absorbent power (especially for sweat) increases.

It can also be noted that none of the compositions according to the prior art are capable of providing performances comparable to the compositions according to the present invention for all three parameters evaluated.

### Example 2 - comparison of parameters relating to the stability of compositions according to the present invention with compositions comprising a filling agent exclusively composed of corn starch

In order to evaluate the stability-related properties of compositions according to the present invention, comparative tests were carried out by analyzing a composition (A1) according to the present invention (comprising in particular a filling agent consisting of corn starch and rice starch) and a composition (A2) comprising an equal quantity of filling agent consisting of corn starch (see Table 3).

**Table 3 - Analysed compositions**

| **Ingredient** | **A1** | **A2** |
|---|---|---|
| Rice starch (Oryza Sativa Starch) | 30.00 | 0.00 |
| Cellulose | 30.00 | 30.00 |
| Corn Starch (Zea Mays Starch) | 24.30 | 54.30 |
| Magnesium carbonate hydroxide | 13.00 | 13.00 |
| Calcium stearate | 2.00 | 2.00 |
| Perfuming composition | 0.70 | 0.70 |

In particular, stability-related parameters were analyzed in accordance with the method ASTM D8328-24. This method relates to the procedures for quantifying the dynamic flow properties of a fixed volume of powder or other bulk solid in relation to its resistance to displacement by a specifically designed blade inside a cylindrical test vessel. This allows resistance to motion to be assessed as a "work done" or measurement of energy. The parameters generated during this test are commonly used in the industry for supporting the design and functioning of powder processing and conveying operations. These parameters can also provide a relative classification or comparison of the flow behavior of different powders, or different batches of the same powder, subjected to similar stress and flow regimes within their respective processing equipment.

From this analysis, it can be noted that powder A2 is less stable than A1: as shown in Table 4, the presence of rice starch in the filling agent leads to an increase in the powder stability index (SI), reducing the agglomeration effect of the particles. It can therefore be stated that the presence of a filling agent consisting of corn starch and rice starch leads to a stabilization of the sample during storage, decreasing its tendency to agglomerate. Furthermore, a decrease in the flow energy is observed, passing from 37.48 mJ for sample A2 to 26.82 mJ for sample A1. As is known, a lower flow energy facilitates the release of the powder from the container and is also linked to greater ease of application by the user. Furthermore, it can be observed that the conditioned bulk density (CBD) values are similar between the two compositions analyzed.

**Table 4 - Experimental results of the comparative tests**

| Sample | BFE (mJ) | SI | CBD (g/ml) |
|---|---|---|---|
| A1 | 26.82 | 1.07 | 0.35 |
| A2 | 37.48 | 1.12 | 0.38 |

## Claims

1. A composition comprising or consisting of:
a) A filling agent consisting of a mixture of rice starch and corn starch, preferably said mixture having a weight ratio of rice starch with respect to corn starch ranging from 0.1 to 10;
b) A glidant agent, said glidant agent being preferably selected from magnesium carbonate hydroxide, magnesium hydroxide, magnesium carbonate, tricalcium phosphate, silica, lauroyl lysine and mixtures thereof, said glidant agent being even more preferably magnesium carbonate hydroxide;
c) An absorbing agent consisting of cellulose, microcrystalline cellulose, methylcellulose, methoxycellulose, hydroxyethylcellulose, hydroxypropylcellulose or mixtures thereof;
d) A hydrophobic agent consisting of a salt of a fatty acid or a mixture of salts of fatty acids, more preferably said hydrophobic agent being a calcium salt of a fatty acid, even more preferably calcium stearate;
e) Optionally, a perfuming agent.

2. The composition according to claim 1, wherein said absorbing agent is present in a concentration by weight with respect to the total weight of the composition ranging from 0.5 to 45%, more preferably from 5 to 40%.

3. The composition according to claim 1, wherein:
• said filling agent is present in a concentration by weight with respect to the total weight of the composition ranging from 50 to 90%;
• said glidant agent is present in a concentration by weight with respect to the total weight of the composition ranging from 5 to 20%;
• said absorbing agent is present in a concentration by weight with respect to the total weight of the composition ranging from 0.5 to 45% and
• said hydrophobic agent is present in a concentration by weight with respect to the total weight of the composition ranging from 0.5 to 5%.

4. The composition according to claim 1, wherein:
• said filling agent is present in a concentration by weight with respect to the total weight of the composition ranging from 50 to 85%;
• said glidant agent is present in a concentration by weight with respect to the total weight of the composition ranging from 10 to 15%, more preferably equal to 13%;
• said absorbing agent is present in a concentration by weight with respect to the total weight of the composition ranging from 0.5 to 40% and
• said hydrophobic agent is present in a concentration by weight with respect to the total weight of the composition ranging from 1 to 3%, more preferably equal to 2%.

5. The composition according to claim 1, wherein:
• said filling agent is present in a concentration by weight with respect to the total weight of the composition ranging from 50 to 60%;
• said glidant agent is present in a concentration by weight with respect to the total weight of the composition ranging from 10 to 15%;
• said absorbing agent is present in a concentration by weight with respect to the total weight of the composition ranging from 25 to 40% and
• said hydrophobic agent is present in a concentration by weight with respect to the total weight of the composition ranging from 0.5 to 5%.

6. The composition according to any one of the previous claims, wherein said absorbing agent is cellulose.

7. The composition according to any one of the previous claims, wherein:
• the weight ratio between rice starch and corn starch ranges from 0.5 to 4, more preferably from 1 to 1.5 and/or
• said glidant agent is magnesium carbonate hydroxide.

8. The composition according to any one of the previous claims, wherein:
• the weight ratio between rice starch and corn starch ranges from 1 to 1.5;
• said glidant agent is magnesium carbonate hydroxide;
• said absorbing agent is cellulose and
• said hydrophobic agent is calcium stearate.

9. The composition according to any one of the previous claims, wherein said composition has a bulk density equal to or less than 0.4 g/ml, more preferably said composition has a bulk density ranging from 0.4 to 0.2 g/ml.

10. The composition according to any one of claims 1 to 9 for topical use as a medicinal product in the prevention and/or treatment of inflammatory skin conditions, more preferably in the prevention and/or treatment of dermatitis, even more preferably contact dermatitis.

11. A non-therapeutic cosmetic method comprising a step for applying to the skin a composition according to any one of claims 1 to 9.

12. A process for producing a composition according to any one of claims 1 to 9 wherein the filling agent, the glidant agent, the absorbing agent, the hydrophobic agent and any further components are mixed together until a homogeneous composition is obtained.
